# EUROPEAN PATENT APPLICATION

(11) **EP 2 078 513 A1**
(43) Date of publication of application: **15.07.2009**
(21) Application number: 09150355.7
(22) Date of filing: 09.01.2009
(51) Int. Cl.: A61F 5/01, A43B 7/26

(54) **Shoe, in particular for the prevention and correction of hallux valgus**

(30) Priority: 11.01.2008 IT TO20080021
(71) Applicant: Orthofoot S.r.l., Verona (IT)
(72) Inventor: Boran, Dario, 37126 Verona (IT); Montanari, Michele, 37014 Castelnuovo del Garda (IT); Angi, Giovanni, 35133 Padova (IT)
(74) Representative: Jorio, Paolo

(57) **Abstract**

A shoe (1) for the correction and prevention of hallux valgus, has an insole and an upper that define a seat for a foot; the seat houses a traction device (11) to maintain lateral pull on the hallux and can be opened/closed by moving a top portion of the upper; the traction device has a traction strap, which is fastened in an adjustable position to the inner surface of the top portion of the upper.

## Description

The present invention relates to a shoe for the prevention, correction and post-operative treatment of all primary and/or secondary deficiency and instability syndromes of the first ray of the foot, and in particular of hallux valgus.

European patent application number EP1772123 describes a shoe that permits constant rehabilitative or preventive exercise when walking, to neutralize and counteract undesirable movements and postures for the pathology of hallux valgus.

Said shoe is in the form of a sandal and comprises a traction device, which is designed to maintain a lateral pull on the hallux towards the inner edge of the shoe, while the head of the first ray of the first metatarsus (generally known as the "bunion") rests against a fixed lateral wall arranged behind the traction device.

The traction device comprises a strap which passes through a transmission ring and that can be pulled by hand and is folded back on itself, for example using Velcro, to adjust the lateral pull on the hallux.

Said solution is not very satisfactory in terms of its aesthetical appearance, as the traction device is visible in the sandal, or from a functional perspective, in that there is a limit to the traction that can be applied by the strap folded back on itself. Said limit is due to the fact that the male and the female tapes of the Velcro can only fit together up to a certain point of the stress exerted on the strap. Beyond that point, the Velcro fastening is not effective or not possible.

The purpose of the present invention is to provide a shoe, in particular for the correction and prevention of hallux valgus, which overcomes the drawbacks described above in a simple and cost-effective manner.

According to the present invention there is provided a shoe, in particular for the correction and prevention of hallux valgus; the shoe comprising:
- a lower supporting surface and an upper, which, between them, define a seat for a foot;
- a lateral supporting wall for a lateral inner portion of the forefoot,
- traction means arranged in front of said lateral supporting wall to maintain lateral pull on the hallux by exerting leverage on said lateral supporting wall; said traction means comprising:
   a) a strap, which has a first end that is fixed with respect to said lower supporting surface and is wound, in use, about said hallux;
   b) releasable fastening means to fasten a second end of said strap in a fixed and adjustable position;
characterized in that said upper comprises a movable portion that can be moved between a closing position, in which it closes an top window of said seat, covers at least a portion of said strap and of the place for said hallux, and is releasably fastened; and an opening position, in which said window is open to access said traction means.

The present invention will now be described with reference to the accompanying drawings, illustrating a non-limiting embodiment thereof, in which:
- figure 1 is a partial perspective view of a preferred embodiment of the shoe, in particular for the correction and prevention of hallux valgus, according to the present invention;
- figure 2 is a different perspective view of the shoe of figure 1, with a traction strap arranged in a different position from that illustrated in figure 1;
- figure 3 is a perspective view of an insole of the shoe of figure 1.

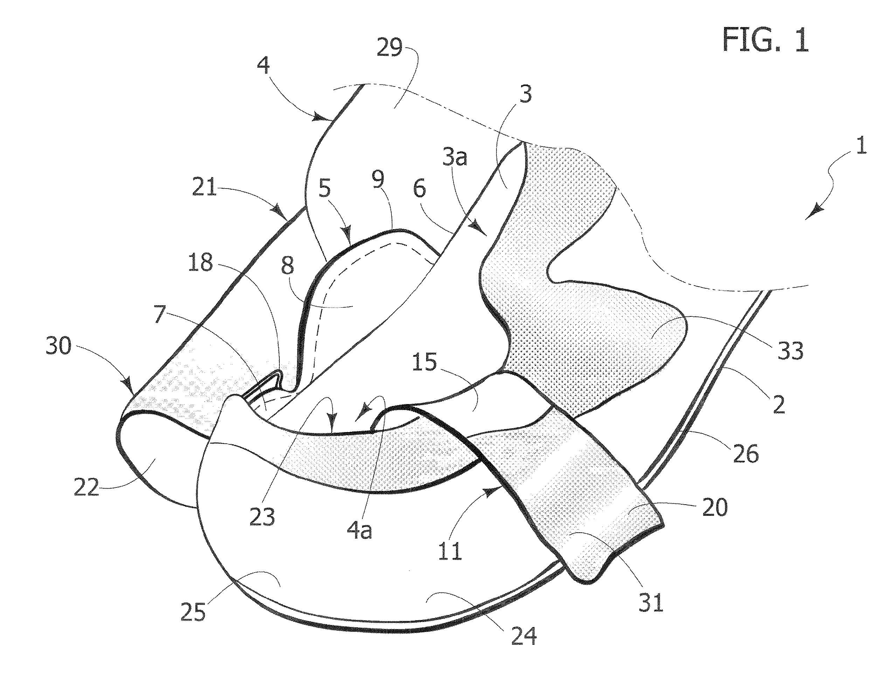

With reference to figure 1, designated as a whole by number 1 is a shoe (partially illustrated) comprising a sole 2 and an insole 3, which is superimposed on the sole 2, is preferably of the type known by the trade name "fussbett", and defines a top surface 3a for supporting the sole of a foot (not illustrated).

The shoe 1 also comprises an upper 4, which is fixed to the sole 2 in a way that is known and not described in detail and defines, with the surface 3a, a seat 4a for the foot.

The seat 4a houses a lateral supporting member 5, defined by a plate or wall substantially parallel to the longitudinal axis of the foot; it is fixed with respect to the sole 2; it is arranged along an lateral inner edge 6 of the shoe 1; it extends upwards with respect to the insole 3; it is of a length such as to extend alongside the whole of the hallux, for example approximately 5-6 centimetres in length; and it comprises a front portion 7 and a rear portion 8. The portion 8 defines a support for a lateral inner portion of the forefoot, defined by the head of the first ray of the first metatarsus, or so-called "bunion".

In particular, the member 5 comprises an inner wall (not illustrated) and a relatively soft outer layer 9. The inner wall has a top indentation between the portions 7 and 8 and constitutes part of an L-shaped body. Said body comprises a base flap arranged between the sole 2 and the insole 3 and fixed to the sole 2. According to an alternative embodiment that is not illustrated, the member 5 has no outer layer 9, with the inner wall arranged between an inner layer and an outer layer of the upper 4.

The inner wall of the member 5 is light in weight, but rigid in a transversal direction in which, in use, the foot exerts the lateral push on the portion 8. For example, the inner wall of the member 5 is made of nylon or glass and carbon fibre, and is of a thickness of between 2 and 3 millimetres. Thus, in use, the portion 8 defines a fulcrum by which to exert leverage on the hallux by means of a traction or return device 11 arranged in the seat 4a opposite the portion 8.

The device 11 is designed to maintain lateral pull on the hallux towards the portion 7, i.e. away from the other toes of the foot. As is known, in a hallux valgus condition the metatarsophalangeal articulation has a bend angle with respect to the normal condition. The combined action of the stiffness of the inner wall of the member 5 and the traction of the device 11 must preferably be such as to reduce said bend angle by at least 50%.

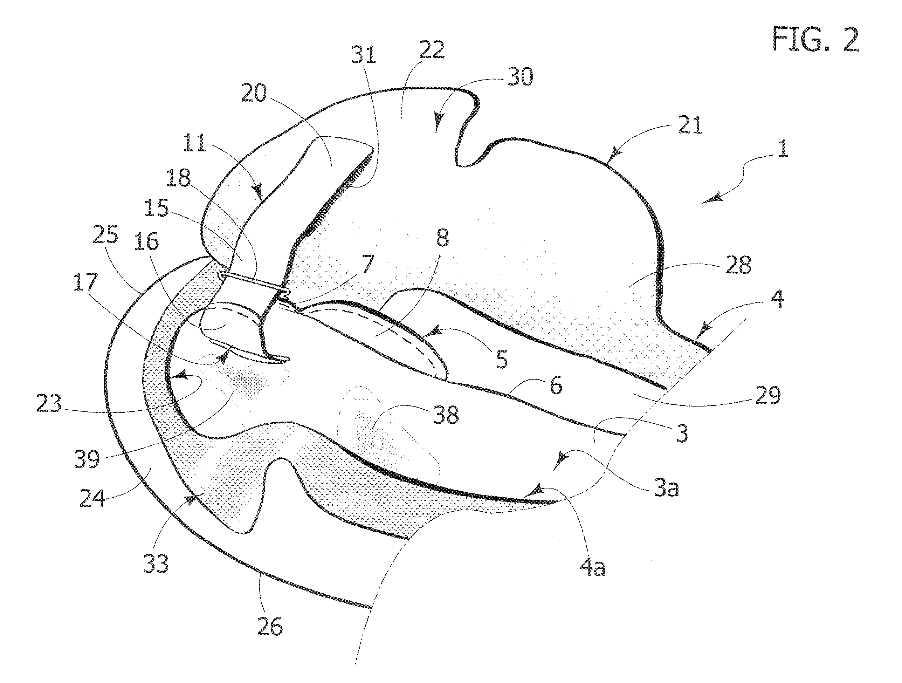

With reference to figure 2, the device 11 comprises a strap or tongue 15, which has one end 16 fixed directly or indirectly to the sole 2, extends through a slit 17 in the insole 3, passes between the hallux and the second toe of the foot, is wound over the hallux, slidingly engages a transmission device 18 fixed with respect to the member 5, and terminates with an end 20 that, in use, is gripped and pulled by hand to tighten the strap 15. The transmission device 18 consists of a ring, which is arranged in the seat 4a between the portion 7 and the upper 4 and, at the bottom, is attached to a fastening tape (not illustrated) fixed to the member 5. As the strap 15 is anchored to the portion 7 by means of the ring 18, in practice the member 5 exerts a pulling force on the hallux through the portion 7, and at the same time exerts a contrasting force on the metatarsus of the foot through the portion 8.

The upper 4 comprises a portion 21 that is flexible and comprises a front strip 22 permanently attached to the sole 2 along the edge 6, alongside the portion 7. The strip 22 is thus foldable in proximity to the edge 6, in an anti-clockwise direction for the right shoe and in a clockwise direction for the left shoe. Thus, the portion 21 can be moved between a closing position (not illustrated), in which the strip 22 closes a top window 23 of the seat 4a to cover the device 11 and the place occupied by the hallux; and an opening position, in which the strip 22 is arranged laterally beyond the edge 6 leaving the window 23 open. In particular, the window 23 is defined by a portion 24 of the upper 4 that extends along the tip 25 and along the outer lateral edge 26 of the shoe 1.

The portion 21 also comprises a strip 28 arranged behind the strip 22 and fixed to a lateral portion 29 of the upper 4, which is fixed to the sole 2 along the edge 6 behind the strip 22 and the front extremity of which is arranged alongside the portion 8.

The strip 22 and the end 20 of the strap 15 are joined by means of a releasable fastening device 31, which defines an adjustable anchor point for the end 20, in order to alter the lateral pull exerted on the hallux. In particular, the device 31 comprises a Velcro fastening between the lower or inner surface 30 of the portion 21 and the end 20.

When the window 23 is open, the strap 15 and the ring 18 are accessible through the window 23, in order to pull the strap 15 and fasten the end 20 to the surface 30. When the window 23 is closed, the portions 21 and 24 are releasably fastened to one another by means of a retaining device 33, which comprises a Velcro fastening between the surface 30 and the top surface of the portion 24. The surface 30 defines the so-called loop fastening, i.e. fabric with a fluffy surface, while the end 30 and the portion 24 bear the so-called hook fastening, i.e. a strip of tape with hooks.

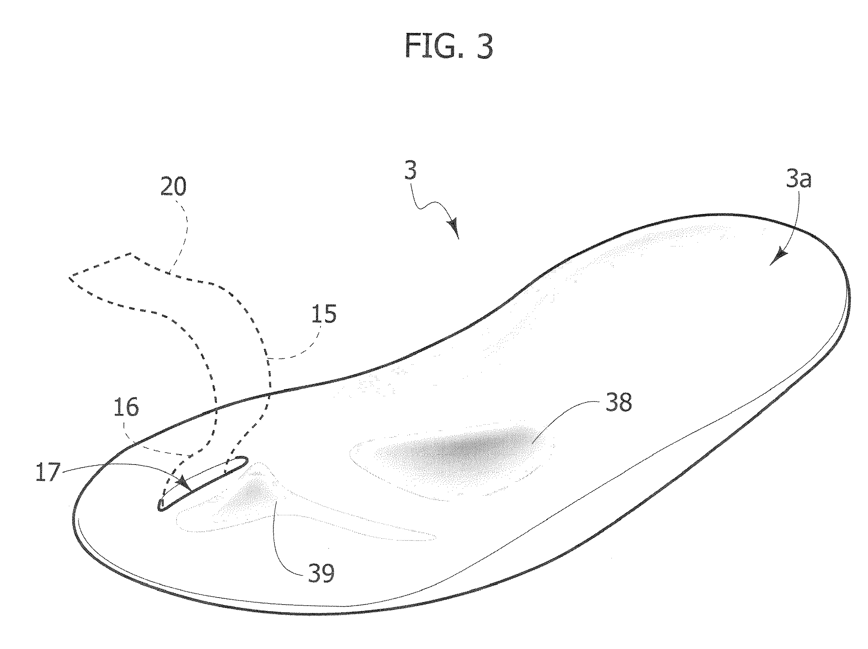

With reference to figure 3, the surface 3a of the insole 3 comprises an arch support 38, in particular a medial arch support, i.e. a supporting projection arranged in correspondence with the arch of the foot to hold or push the arch upwards when walking, in order to counter flat-footedness caused by the hallux valgus condition. Moreover, the surface 3a of the insole 3 comprises a retrotarsal support 39, commonly referred to as an "olive sole support", i.e. a supporting projection arranged immediately before the metatarsal heads of the second, third and fourth metatarsal bones. The retrotarsal support 39 enables the weight of the foot to be loaded not only on the hallux, but also on the metatarsal rays of the other toes.

By distributing the weight on the various toes by means of the retrotarsal support 39 and maintaining the arch of the foot raised by means of the arch support 38, when walking the weight sustained by the foot is partly discharged towards the outer edge: the reduced load on the inner side of the foot increases the effect of the lateral pull exerted by the device 11, facilitating the correction of hallux valgus.

Moreover, the rear part of the upper 4 defines a lateral shell on both sides of the heel of the foot, to block the heel laterally and prevent the foot from moving sideways when walking, providing a further supporting point for the leverage exerted by the device 11, in addition to the portion 8 of the member 5.

In accordance with the above description, the portion 21 of the upper 4 allows the hallux and at least the lower portion of the strap 15 to be covered, with obvious advantages in terms of aesthetical appearance, and the device 11 is easily accessible in order to exert the pulling force on the hallux in a simple and effective manner, once the window 23 is open.

Moreover, since the strap 15 is attached to the portion 21 the tension of the device 11 can be determined simply and quickly, a relatively long strap 15 can be used, which is easy to grip and does not get in the way of the toes, and the male and female Velcro fastenings always fit together properly.

Moreover, during the closing of the window 23 the strip 22 is folded back on the window 23 and pulled towards the edge 26 until joining the Velcro of the device 33, so that the end 20 is pulled with the strip 22 and the strap 15 is tightened further.

Moreover, the Velcro of the device 33 allows the closing position of the portion 21 on the portion 24 to be adjusted according to the size of the foot and enables the window 23 to be closed quickly.

It is apparent that the arch support 38 and the retrotarsal support 39 work together with the member 5 and the device 11, to actively prevent or correct incorrect bending of the hallux. When walking the components of the articulation of the hallux are realigned during the movement, preventing any consequences of the hallux valgus condition (metatarsalgie of other toes, postural alterations such as genu valgum, lumbosacral contractures, etc.).

Lastly, from the above description, it is clear that modifications and variations may be made to the shoe 1 described herein without departing from the scope of the present invention as set forth in the appended claims.

In particular, the upper 4 could be incomplete, for example defining a shoe in the form of a sandal or slipper, always with at least a portion of the strap 15 arranged beneath the upper.

Moreover, the ring 18 could be replaced with a different fastening element, and/or be integrated into the upper 4; and/or the end 20 could protrude outside the seat 4a when the window 23 is closed; and/or the devices 31 and 33 could be of a type other than the Velcro type.

## Claims

1. Shoe (1), in particular for the prevention and correction of hallux valgus; the shoe comprising:
- a lower supporting surface (3a) and an upper (4), which, between them, define a seat (4a) for a foot;
- a lateral supporting wall (8) for a lateral inner portion of the forefoot,
- traction means (11) arranged in front of said lateral supporting wall (8) to maintain lateral pull on the hallux by exerting leverage on said lateral supporting wall (8); said traction means (11) comprising:
a) a strap (15), which has a first end (16) that is fixed with respect to said lower supporting surface (3a) and is wound, in use, about said hallux;
b) releasable fastening means(31) to fasten a second end (20) of said strap (15) in a fixed and adjustable position;
**characterized in that** said upper (4) comprises a movable portion (22) that can be moved between a closing position, in which it closes a top window (23) of said seat (4a), covers at least a portion of said strap (15) and of the place for said hallux, and is releasably fastened; and an opening position, in which said window (23) is open to access said traction means (11).

2. Shoe according to claim 1, **characterized in that** said traction means (11) comprise transmission means (18) slidingly engaged by said strap (15), and **in that** said fastening means (31) fasten said second end (20) to said movable portion (22).

3. Shoe according to claim 2, **characterized in that** said fastening means (31) fasten said second end (20) directly to a surface (30) facing towards said seat (4a).

4. Shoe according to claim 3, **characterized in that** said fastening means (31) comprise a Velcro fastening.

5. Shoe according to any one of the previous claims,
**characterized in that** said movable portion (22) is foldable in proximity to the lateral inner edge (6) of the shoe (1) between said opening and closing positions.

6. Shoe according to any one of the previous claims,
**characterized in that** said movable portion (22), when in the closing position, is fastened to a further portion (24) of the upper by means of a Velcro fastening.

7. Shoe according to any one of the previous claims,
**characterized in that** said lower supporting surface (3a) comprises an arch support.

8. Shoe according to any one of the previous claims,
**characterized in that** said lower supporting surface (3a) comprises a retrotarsal support.
